# EUROPÄISCHE PATENTSCHRIFT

(11) **EP 2 498 627 B1**
(45) Veröffentlichungstag und Bekanntmachung des Hinweises auf die Patenterteilung: **13.08.2014**
(21) Anmeldenummer: 10771064.2
(22) Anmeldetag: 11.10.2010
(51) Int. Cl.: A23L 1/30, A61K 8/99, A61K 35/74, A61Q 11/00, A61P 1/02, C12R 1/225

(54) **GESUNDHEITSFÖRDERLICHE ZUBEREITUNG UND HERSTELLVERFAHREN**
HEALTH-BENEFICIAL PREPARATION AND PRODUCTION METHOD
PRÉPARATION FAVORABLE À LA SANTÉ ET PROCÉDÉ DE FABRICATION

(30) Priorität: 10.11.2009 EP 09014070
(43) Veröffentlichungstag der Anmeldung: 19.09.2012
(73) Patentinhaber: BASF SE, 67056 Ludwigshafen (DE)
(72) Erfinder: COOPER, Bryan, 68199 Mannheim (DE)
(86) Internationale Anmeldenummer: PCT/EP2010/065180
(87) Internationale Veröffentlichungsnummer: WO 2011/057872

(56) Entgegenhaltungen:
- WO-A1-2006/027265
- DATABASE FSTA [Online] INTERNATIONAL FOOD INFORMATION SERVICE (IFIS), FRANkFURT-MAIN, DE; MORTAZAVIAN A M ET AL: "Combined effects of temperature-related variables on the viability of probiotic micro-organisms in yogurt.", XP002617641, Database accession no. FS-2007-02-Pl0368 & MORTAZAVIAN A. M. ET AL: "Combined effects of temperature-related variables on the viability of probiotic micro-organisms in yogurt.", AUSTRALIAN JOURNAL OF DAIRY TECHNOLOGY, Bd. 61, Nr. 3, 2006, Seiten 248-252,

## Beschreibung

Die Erfindung betrifft das Gebiet gesundheitsförderlicher Zubereitungen und ihrer Herstellverfahren.

Streptococcus mutans spielt eine zentrale Rolle bei der Entwicklung von Karies. Das Bakterium setzt vergärbare Zucker um zu organischen Säuren und erzeugt so eine saure Mikroumgebung. Die organischen Säuren können den Zahnschmelz demineralisieren und so die Entwicklung kariotischer Läsionen bewirken oder fördern. Darüber hinaus erzeugt S. mutans eine nichtwasserlösliche Glukanmatrix. Diese unterstützt die Bildung und Anhaftung von Plaque sowie die Anhaftung von S. mutans an der Zahnoberfläche. Ferner hat sich gezeigt, dass auch weitere Bakterien häufig in kariotischen Läsionen gefunden werden, jedoch stets zusammen mit S. mutans. Dementsprechend gilt S. mutans gegenwärtig als notwendig für die Entwicklung kariotischer Läsionen.

Es besteht ein ständiger Bedarf an Mitteln, mit denen der Bildung kariotischer Läsionen vorgebeugt werden kann. In diesem Zusammenhang bezweckt die Erfindung Mittel anzugeben, mit denen auf S. mutans eingewirkt werden kann, um die Bildung kariotischer Läsionen zu vermeiden, aufzuhalten oder zu verlangsamen. Die Erfindung bezweckt ebenfalls, Herstellverfahren für entsprechende Mittel anzugeben.

Zum Bekämpfen von kariotischen Läsionen, insbesondere um ihr Auftreten zu vermeiden oder zumindest zu verzögern, sind in der Vergangenheit eine ganze Reihe an Mitteln erprobt worden. Dem Fachmann bekannt sind insbesondere übliche chemische Mittel zum Bekämpfen von Karies und Karies-assoziierten Mikroorganismen, wie sie in Zahnpasten und/oder Mundwässern und/oder anderen Zahnpflegeprodukten Verwendung finden. Darüber hinaus ist aber auch versucht worden, Karies-assoziierte Mikroorganismen durch andere Mikroorganismen oder deren Produkte zu bekämpfen, insbesondere ihre Menge auf Zähnen oder in der Mundhöhle zu verringern oder ihre Kariogenizität anderweitig zu beeinflussen. Ein solcher Ansatz ist beispielsweise beschrieben in der WO 2006/027265 A1. Nachteilig daran ist jedoch, dass die Verwendung lebender Mikroorganismen in Mundpflegeprodukten von Verbrauchern häufig abgelehnt oder von Gesetzgebern gänzlich verboten wird. Zudem können lebende Mikroorganismen durch Stoffwechselprodukte den Geschmack und/oder das Aussehen eines sie enthaltenden Produktes beeinflussen; diese Beeinflussung mag nicht für jedes Produkt zum Bekämpfen kariotischer Läsionen gewünscht sein. Zudem wird durch den Einsatz lebender Mikroorganismen in zum Bekämpfen kariotischer Läsionen bestimmter Produkte deren Haltbarkeit begrenzt.

Ganz allgemein wird deshalb auf verschiedentlichen technischen Gebieten versucht, anstelle lebender Mikroorganismen stoffwechselinaktive Mikroorganismen, insbesondere lyophilisierte Mikroorganismen zu verwenden. Dabei ist jedoch problematisch, dass solche Mikroorganismen unter für sie geeigneten Bedingungen wieder stoffwechselaktiv werden können, beispielsweise wenn sie in ein zuträgliches wässriges Medium geraten. Die Verwendung stoffwechselinaktiver Mikroorganismen zieht dementsprechend starke Beschränkungen der möglichen Zusammensetzung eines sie enthaltenden Produktes nach sich.

Es ist deshalb auf anderen technischen Gebieten versucht worden, anstelle lebender oder stoffwechselaktiver Mikroorganismen Produkte mit abgetöteten Mikroorganismen herzustellen. So zeigt beispielsweise die WO 01/95741 A1 ein Lebensmittel zum Fördern des Darmgleichgewichtes, wobei das Lebensmittel nicht-lebensfähige Lactobacillen enthält. Die Lactobacillen können durch Wärmebehandlung nicht-lebensfähig gemacht werden, beispielsweise durch Pasteurisieren oder Sterilisieren. In diesem Dokument wird jedoch auch auf die Gefahr hingewiesen, durch die Wärmebehandlung einige ansonsten erreichbare gesundheitsfördernde Effekte von Mikroorganismen zu verlieren. Zwar heißt es im Dokument, diese gesundheitsfördernden Effekte könnten "selektiv" beseitigt werden, tatsächlich gibt das Dokument jedoch keine Lehre dahingehend, wie der Verlust eines gewünschten gesundheitsfördernden Merkmals bei einer Wärmebehandlung von Mikroorganismen vermieden werden könnte. In Anbetracht dieses Dokuments kann der Fachmann also weder vorhersagen noch sinnvolle Vermutungen darüber anstellen, welche gesundheitsfördernden Effekte durch eine Wärmebehandlung von Mikroorganismen verloren gehen und welche nicht. Insbesondere ist nicht vorhersehbar oder abschätzbar, ob ein antikariogener Effekt durch Wärmebehandlung verloren geht.

Es war deshalb die Aufgabe der vorliegenden Erfindung, Mittel zum Bekämpfen kariotischer Läsionen anzugeben, insbesondere Mittel zum Vermeiden oder Verzögern der Bildung kariotischer Läsionen. Die Mittel sollten insbesondere geeignet sein, den gewünschten Effekt sicher herbeizuführen. Sie sollten einfach und preisgünstig herstellbar sein und die oben beschriebenen Nachteile nach Möglichkeit vermeiden oder nur in geringem Maße in Kauf nehmen.

Erfindungsgemäß wird deshalb ein Verfahren angegeben zum Herstellen einer nichtlebenden Lactobacillus-Zubereitung mit spezifischer Bindungsfähigkeit für Streptococcus mutans, umfassend die Schritte:
i) Erwärmen einer Suspension von Zellen eines Lactobacillus oder einer Mischung von Lactobacillen, mit spezifischer Bindungsfähigkeit an Streptococcus mutans, wobei das spezifische Binden
   a) widerstandsfähig gegen Wärmebehandlung und/oder
   b) widerstandsfähig gegen Proteasebehandlung und/oder
   c) calziumabhängig ist und/oder
   d) in einem pH-Bereich zwischen 4,5 und 8,5 stattfindet, und/oder
   e) in Gegenwart von Speichel erfolgt,
   von einer Ausgangstemperatur unter 40°C auf eine Pasteurisierungstemperatur von 75 bis 85°C mit einer Temperaturänderung von 0,5 bis 2°C/min.,
ii) Halten der erwärmten Suspension auf der Pasteurisierungstemperatur über einen Zeitraum von 20 bis 40 min.,
iii) Abkühlen der in Schritt ii) gehaltenen Suspension auf eine Endtemperatur unter 40°C mit einer Temperaturänderung von 0,5 bis 2°C/min.

Derartige Lactobacillen sind offenbart in WO 2006/027265 A1, deren Inhalt zum Zwecke der Offenbarung der vorliegenden Erfindung vollständig in Bezug genommen wird.

Überraschenderweise hat sich gezeigt, dass mit den erfindungsgemäß ausgewählten Lactobacillen durch schonende Pasteurisierung entsprechend den Schritten des erfindungsgemäßen Verfahrens das Herstellen einer Zubereitung gelingt, die vollständig oder im Wesentlichen frei ist von lebenden, stoffwechselaktiven Lactobacillus-Mikroorganismen und dennoch eine spezifische Bindungsfähigkeit für Streptococcus mutans aufweist. Eine erfindungsgemäß hergestellte Zubereitung ist deshalb geeignet zum Ausüben eines antikariogenen Effekts und dementsprechend nützlich als Mittel zum Verhindern und/oder Behandeln von Karies. Die Begriffe "Karies" und "kariotischen Läsion" werden im Rahmen dieses Dokuments austauschbar gebraucht und bezeichnen eine chronische Infektionskrankheit, die sich durch Bildung erweichter Stellen auf bzw. in einem Zahn auszeichnet und fortschreitend zum Tod des Zahns führt. Karies kann durch bekannte Verfahren diagnostiziert werden; der Fachmann kann dazu beispielsweise zurückgreifen auf die Veröffentlichung Angmar-Mansson und ten Bosch, Adv. Dent. Res. 7 (1993), 70 bis 79.

Im Sinne dieser Erfindung schließt der Begriff "Bekämpfen von Karies" bzw. "Bekämpfen von kariotischen Läsionen" die Kariesprophylaxe mit ein. Auch Personen, deren Mundhöhle frei von S. mutans sein sollte, können also von den erfindungsgemäß hergestellten Zubereitungen profitieren, indem diese Zubereitungen zufällig eingetragene Zellen von S. mutans binden und so deren Beseitigung erleichtern.

Im Sinne dieser Erfindung bedeutet der Ausdruck "Behandlung von Karies" auch die Verabreichung der erfindungsgemäß hergestellten Zubereitungen an eine an Karies erkrankte Person zum Zwecke des Verringerns der Menge an S. mutans-Zellen und ggf. zum vollständigen Beseitigen von S. mutans aus dem Mund, insbesondere der gesamten Mundhöhle einschließlich der Zähne und Zahnzwischenräume.

Die erfindungsgemäß ausgewählten Mikroorganismen sind im Hinblick auf ihre Fähigkeit zum Binden von S. mutans sehr hitzestabil. Dies ermöglicht es, den in der WO 2006/027265 A1 für lebende Lactobacillus-Zellen beschriebenen gesundheitsfördernden Effekt des Bindens an S. mutans auch nach erfindungsgemäßer Pasteurisierung aufrechtzuerhalten und nicht etwa zu verlieren. Dies ist besonders ungewöhnlich, da für Mikroorganismen grundsätzlich zu erwarten gewesen ist, dass bei Pasteurisierung ein erheblicher Anteil beispielsweise ihrer Proteine denaturiert und andere Zellbestandteile aufgelöst oder beschädigt werden, so dass gesundheitsförderliche Effekte von Mikroorganismen nach dem Pasteurisieren gewöhnlich verloren gehen. So zeigt ja auch die oben erwähnte WO 01/95741 A1 lediglich, dass es für bestimmte Lactobacillen möglich ist, einen gesundheitsfördernden Effekt auf die Darmflora trotz Pasteurisierung aufrechtzuerhalten. Allerdings ist die Mundhöhle nach Struktur, Inhalt und Beanspruchung völlig anders beschaffen als der Darm, so dass der Fachmann die Erkenntnisse des letztgenannten Dokuments nicht auf die vorliegende Erfindung übertragen konnte.

Gemäß einem weiteren Aspekt der Erfindung wird dementsprechend eine Lactobacillus-Zubereitung mit spezifischer Bindungsfähigkeit für Streptococcus mutans angegeben, die erhältlich oder erhalten ist durch ein erfindungsgemäßes Herstellverfahren. Eine solche Zubereitung verwirklicht die oben beschriebenen Vorteile des erfindungsgemäßen Herstellverfahrens, insbesondere ist sie geeignet zum Herstellen eines antikariogenen Produktes zur Anwendung am menschlichen Körper.

Vorzugsweise sind die Lactobacillus-Zellen ausgewählt aus Zellen von Stämmen von Lactobacillus paracasei, Lactobacillus rhamnosus oder einer Mischung derselben. Das erfindungsgemäße Herstellverfahren kann also mit einer Suspension einer Reinkultur eines Lactobacillus-Stammes, insbesondere eines Stammes von Lactobacillus paracasei bzw. Lactobacillus rhamnosus, aber auch mit einer Mischung von zwei, drei, vier, fünf, sechs, sieben oder mehr Stämmen ausgeführt werden. Für das erfindungsgemäße Verfahren besonders bevorzugt sind die in der WO 2006/027265 A1 genannten Lactobacillus-Arten und Stämme, insbesondere solche mit einer DSMZ-Nummer DSMZ 16667, DSMZ 16668, DSMZ 16669, DSMZ 16670, DSMZ 16671, DSMZ 16672 und DSMZ 16673, jeweils hinterlegt bei der Deutschen Sammlung für Mikroorganismen und Zellkulturen, Mascheroder Weg 1b, Braunschweig, Deutschland, am 26. August 2004. Soweit im Rahmen dieser Erfindung von Lactobacillus-Zellen gesprochen wird, sind damit zumindest vorzugsweise auch Zellen der soeben genannten Stämme und Mischungen von zwei, drei, vier, fünf, sechs oder sieben der genannten Stämme gemeint. Der Fachmann versteht, dass anstelle oder zusätzlich zu Zellen eines der genannten Stämme auch eine Mutante oder eine abgeleitete Zelllinie im erfindungsgemäßen Verfahren eingesetzt werden kann, wobei die Mutante oder abgeleitete Zelllinien die Fähigkeit zum spezifischen Binden an S. mutans bewahrt hat.

Die Lactobacillus-Zellen besitzen vorzugsweise eine spezifische Bindungsfähigkeit für Streptococcus mutans Serotyp c (DSMZ 20523) und/oder Serotyp e (NCTC 10923) und/oder Serotyp F (NCTC 11060).

Eine Mutante, insbesondere eine Mutante eines der vorgenannten Lactobacillus-Stämme, besitzt eine oder mehrere dauerhafte vererbliche Veränderungen, üblicherweise seine Nukleinsäure(n). Solche Veränderungen schließen üblicherweise Punktmutationen wie Transitionen oder Transversionen, aber auch Deletionen, Insertionen und Additionen einer oder mehrerer Basen einer Nukleinsäure mit ein, wodurch die Nukleinsäure geändert wird und eine von der Norm abweichende Genexpression und/oder Transkription und/oder Translation bzw. Inaktivierung von Genprodukten bewirkt wird. Mutationen können spontan auftreten oder durch Einwirken von Agenzien wie beispielsweise Chemikalien oder Bestrahlung ausgelöst werden. Verfahren zum Selektieren und Gewinnen von Mutanten und abgeleiteten Zelllinien sind beispielsweise beschrieben in Sambrook, "Molekular cloning, a laboratory manual", Cold Spring Harbour Laboratory, NY, (2001) sowie Ausubel, "Current protocols in molecular biology", Green Publishing Associates and Wiley Interscience NY, (1989). Weitere Angaben entnimmt der Fachmann der WO 2006/027265 A1.

Im Sinne dieser Erfindung bedeutet "spezifisches Binden" bzw. "spezifische Bindungsfähigkeit" für Streptococcus mutans, dass die erfindungsgemäß verwendeten Lactobacillus-Zellen bzw. die erfindungsgemäß pasteurisierten Zellen an S. mutans binden, an die meisten oder alle der übrigen in der Mundhöhle gewöhnlich in bedeutsamen Mengen vorkommenden Mikroorganismen jedoch nicht binden. Vorzugsweise binden die erfindungsgemäß ausgewählten Mikroorganismen nicht an Zellen von Streptococcus salivarius, Streptococcus salivarius thermophilus, Streptococcus oralis, Streptococcus mitis und/oder Streptococcus sanguinis. Besonders bevorzugt binden die erfindungsgemäß verwendeten Lactobacillus-Zellen nicht an Streptococcus salivarius ssp. thermophilus API 50 CH (Biomerieux, France), Streptococcus oralis DSMZ 20066, Streptococcus oralis DSMZ 20395, Streptococcus oralis DSMZ 20627, Streptococcus mitis DSMZ 12643 und/oder Streptococcus sanguinis DSMZ 20567. Ebenfalls bevorzugt ist es, wenn die erfindungsgemäß verwendeten Lactobacillus-Zellen nicht an Bakterien anderer Genera als Streptococcus binden, also beispielsweise nicht an Zellen des Genus Staphylococcus binden. Besonders bevorzugt binden sie nicht an Staphylococcus epidermidis DSMZ 1798 und/oder Stapyhlococcus epidermidis DSMZ 20044. Zum Testen der Bindungsfähigkeit geht der Fachmann wie in WO 2006/027265 A1 beschrieben vor, indem er die genannten Bakterien mit den erfindungsgemäß ausgewählten Lactobacillen bzw. deren erfindungsgemäß pasteurisierten Reste in einem volumetrischen Verhältnis von 3:1 mischt und eine eventuell von Lactobacillus ausgelöste Aggregation beobachtet. Ein entsprechendes Verfahren ist in der genannten WO-Schrift in Beispiel 3 beschrieben.

Dementsprechend bevorzugt ist ein erfindungsgemäßes Verfahren, bei dem Zellen eines oder mehrerer der bevorzugten Lactobacillus-Stämme, insbesondere L. paracasei oder L. rhamnosus und vorzugsweise einer oder mehrere der Stämme DSMZ 16667, DSMZ 16668, DSMZ 16669, DSMZ 16670, DSMZ 16671, DSMZ 16672 und DSMZ 16673 und besonders bevorzugt zumindest DSM 16671, und/oder einer wie oben beschriebenen Mutante oder abgeleiteten Zelllinie, in einem ersten Schritt in einer Suspension erwärmt werden von einer Ausgangstemperatur unter 40°C auf eine Pasteurisierungstemperatur von 75 bis 85°C mit einer Temperaturänderung von 0,5 bis 2°C, in einem zweiten Schritt 20 bis 40 min lang auf der Pasteurisierungstemperatur gehalten werden (Pasteurisierungszeit), und bei dem die Suspension in einem anschließenden dritten Schritt auf eine Endtemperatur unter 40°C mit einer Temperaturänderung von 0,5 bis 2°C abgekühlt wird.

Die Pasteurisierungstemperatur im Schritt i) beträgt vorzugsweise 78 bis 80°C, besonders bevorzugt 80°C. Bei diesen Pasteurisierungstemperaturen gelingt eine zügige und entsprechend energiesparende und dennoch sichere Abtötung der erfindungsgemäß ausgewählten Lactobacillus-Zellen bei gleichzeitig geringem Verlust an Bindungsfähigkeit im Vergleich zu einer nicht pasteurisierten Kultur lebender Lactobacillus-Zellen.

Die Pasteurisierungszeit beträgt vorzugsweise 25 bis 35 min. und besonders bevorzugt 30 min. Es hat sich herausgestellt, dass insbesondere bei einer Pasteurisierungstemperatur von 78 bis 80°C (bevorzugt von 80°C), ein rasches Abtöten der erfindungsgemäß ausgewählten Lactobacillus-Zellen bei geringem Verlust an S. mutans-Bindungsfähigkeit im Vergleich zu einer lebenden Kultur der Lactobacillus-Zellen möglich ist.

Ebenfalls bevorzugt ist es, wenn die Temperaturänderung in Schritt i) oder iii) 0,8 bis 1,2°C pro min. beträgt, vorzugsweise 1°C pro min. Auf diese Weise kann die Aufheizzeit bis zum Erreichen der Pasteurisierungstemperatur vorteilhaft kurz gehalten werden, so dass Energie eingespart werden kann, ohne dass dadurch das Abtöten der Lactobacillus-Zellen gefährdet oder ein starker Verlust an Bindungsfähigkeit für S. mutans in Kauf genommen werden müsste.

Insbesondere bevorzugt ist also ein erfindungsgemäßes Verfahren, bei dem Zellen eines oder mehrerer der bevorzugten Lactobacillus-Stämme L. paracasei oder L. rhamnosus, vorzugsweise einer oder mehrere der Stämme DSMZ 16667, DSMZ 16668, DSMZ 16669, DSMZ 16670, DSMZ 16671, DSMZ 16672 und DSMZ 16673 und besonders bevorzugt zumindest DSM 16671, und/oder einer wie oben beschriebenen Mutante oder abgeleiteten Zelllinie, in einem ersten Schritt in einer Suspension erwärmt werden von einer Ausgangstemperatur unter 40°C auf eine Pasteurisierungstemperatur von 78 bis 80°C, vorzugsweise 80°C, mit einer Temperaturänderung von 0,8 bis 1,2°C, vorzugsweise von 1°C, in einem zweiten Schritt 25 bis 35 min und bevorzugt 30 min lang auf der Pasteurisierungstemperatur gehalten werden (Pasteurisierungszeit), und bei dem die Suspension in einem anschließenden dritten Schritt auf eine Endtemperatur unter 40°C mit einer Temperaturänderung von 0,8 bis 1,2°C, vorzugsweise von 1°C, abgekühlt wird.

Die erfindungsgemäß pasteurisierten Zellen befinden sich vorzugsweise in einer postexponentiellen Wachstumsphase. Besonders bevorzugt werden in Schritt i) des erfindungsgemäßen Herstellverfahrens Zellen aus einem Kultivierungsmedium verwendet, das zunächst ein exponentielles Wachstum ermöglichte, bei dem jedoch die Glucose-Konzentration auf einen Wert von maximal 1 mM Glucose gefallen ist. Dabei ist es bevorzugt, solche Zellen zu verwenden, dessen Glucose-Konzentration seit nicht mehr als 1 h auf einen Wert von maximal 1 mM Glucose gefallen ist, da ansonsten die Fähigkeit des Produktes des erfindungsgemäßen Herstellverfahrens, also des Ergebnisses von Schritt iii), gegebenenfalls des Waschschrittes oder der Sprühtrocknung wie unten beschrieben, zum spezifischen Binden an Streptococcus mutans beginnt verloren zu gehen.

Das erfindungsgemäße Verfahren umfasst bevorzugt ferner einen Waschschritt, bei dem die in Schritt (iii) erhaltene Suspension zum Aufkonzentrieren der pasteurisierten Zellen zentrifugiert wird, die so erhaltenen konzentrierten Zellen mit Wasser versetzt und erneut zum Aufkonzentrieren der Zellen erneut zentrifugiert werden. Die gewonnenen konzentrierten Zellen liegen als Substanz mit einem Trockengehalt von 10 bis 30 Gew.-% der Substanz vor, vorzugsweise von 18 bis 22 Gew.-%.

Erfindungsgemäß besonders bevorzugt ist ein solches Verfahren, das neben den Schritten i) bis iii) (insbesondere angewendet auf einen oder eine Mischung von zwei, drei, vier, fünf, sechs oder sieben der Lactobacillus-Stämme DSM 16667, DSM 16668, DSM 16669, DSM 16670, DSM 16671, DSM 16672 und DSM 16673 oder einer wie oben beschriebenen Mutante oder abgeleiteten Zelllinie oder einem Stamm von L. paracasei oder L. rhamnosus) und gegebenenfalls Waschen folgende Schritte umfasst:
iv) Versetzen der in Schritt iii) erhaltenen und vorzugsweise gewaschenen Suspension mit einem Träger zum Herstellen einer Sprühtrocknungs-Mischung, wobei der Träger ausgewählt ist aus Alkali- und Erdalkali-Sulfaten, vorzugsweise Natrium-, Kaliumoder Calciumsulfat, und Mischungen zweier oder mehrerer dieser Sulfate, und wobei die Menge an Träger so gewählt wird, dass der Trockengehalt der Sprühtrocknungsmischung 10 bis 30 Gew.-% beträgt, und
v) Sprühtrocknen der ein Schritt iv) erhaltenen Sprühtrocknungs-Mischung.

Sprühtrocknung setzt das zu trocknende Gut sehr harschen Bedingungen und insbesondere Temperaturen von beispielsweise 70°C bis 200°C aus. Selbst von den erfindungsgemäß ausgewählten Lactobacillus-Zellen war nicht bekannt, dass sie den beim Sprühtrocknen auftretenden harschen Bedingungen ohne signifikanten Verlust an spezifischer Bindungsfähigkeit für S. mutans standhalten. Hinzukommt, dass bei den erfindungsgemäß hohen Anteilen an Träger in Schritt iv) zu erwarten gewesen wäre, dass die für die Bindung an S. mutans verantwortlichen Oberflächenstrukturen von Lactobacillus denaturiert oder anderweitig beschädigt werden. Es hat sich nun überraschend herausgestellt, dass unter den gewählten Bedingungen eine Sprühtrocknung der in Schritt iii) erhaltenen Suspension ohne wesentlichen Verlust an Bindungsfähigkeit für S. mutans möglich ist.

Einige Vorteile des erfindungsgemäßen Sprühtrocknungsverfahrens und der so hergestellten erfindungsgemäßen sprühgetrockneten Zubereitung verdienen besondere Beachtung: Anders als durch bloßes Pasteurisieren erlaubt es die erfindungsgemäße Sprühtrocknung, eine nicht klebrige oder hygroskopische Zubereitung zu erhalten. Hygrospkopisch bedeutet in diesem Zusammenhang, dass bei einer offenen Lagerung über einen Zeitraum von 12 Monaten bei einer Temperatur von 20 °C und einer relativen Luftfeuchtigkeit von 50% das Gewicht der Zubereitung um nicht mehr als 2% zunimmt. Die so hergestellte erfindungsgemäße Zubereitung ist deshalb besonders geeignet zur Weiterverarbeitung in trockenen Produkten; sie wird die Eigenschaften dieser Produkte nicht durch das Anziehen von Wasser oder eine besondere Klebrigkeit nachteilig beeinflussen.

Ferner ermöglicht das erfindungsgemäße Herstellverfahren das Herstellen einer erfindungsgemäßen Zubereitung, die frei fließend ist. Partikelförmige, frei fließende Zubereitungen sind besonders einfach lagerbar und verarbeitbar, sie können in unterschiedlichste Produkte eingearbeitet und dabei sehr präzise dosiert werden. Durch das erfindungsgemäße Sprühtrocknungsverfahren erhaltene erfindungsgemäße Zubereitungen können somit in einem wirtschaftlich bedeutsamen Ausmaße in einem viel größeren Produktspektrum eingearbeitet werden als die nach Schritt iii) unmittelbar erhaltene Suspension.

Als Träger in Schritt iv) besonders bevorzugt ist Natriumsulfat. Vorzugsweise beträgt die Menge an Träger, besonders bevorzugt an Natriumsulfat, das 4-fache der Trockenmasse der aus Schritt iii) eingesetzten Suspension. Die in Schritt v) zum Sprühtrocknen eingesetzte Sprühtrockungsmischung hat dann einen Anteil von etwa 20 Gew.-% an Gesamt-Trockensubstanz.

Im Schritt v) erfolgt die Sprühtrocknung vorzugsweise bei einer Gaseintrittstemperatur eines zur Trocknung eingesetzten Sprühtrocknungsgases von 180 bis 250°C und einer Gasaustrittstemperatur aus dem zur Trocknung vorgesehenen Bereich, üblicherweise einem Sprühturm, von 70 bis 100°C, vorzugsweise 85°C, wobei die durchschnittliche Verweilzeit des zu trocknenden Gutes im Einwirkbereich des Sprühtrocknungsgases 10 bis 60 Sekunden, vorzugsweise 20 bis 40 Sekunden beträgt . Der Fachmann kann sich im Weiteren für die Sprühtrocknung an den Angaben der WO 01/25411 A1 orientieren. Dieses Dokument beschreibt allgemein geeignete Sprühtrocknungsverfahren zum Herstellen sprühgetrockneter Enzymprodukte. Überraschenderweise hat sich nunmehr herausgestellt, dass auch bei den erfindungsgemäß bevorzugt höheren Temperaturen eine Sprühtrocknung ohne wesentlichen Verlust an Bindungsfähigkeit für S. mutans möglich ist. Dies war auf Grundlage der WO 01/25411 A1 nicht zu erwarten gewesen, insbesondere weil darin kein einziges Beispiel einer funktionierenden Sprühtrocknung nach Pasteurisierung beschrieben ist und das Dokument hinsichtlich der Geeignetheit der Sprühtrocknung für eine angeblich sehr breite Klasse von Enzymen aus fachmännischer Sicht spekulativ ist.

Ein solches erfindungsgemäßes Verfahren ist daher besonders bevorzugt, bei dem:
(i) Zellen eines oder mehrerer der bevorzugten Lactobacillus-Stämme L. paracasei oder L. rhamnosus, vorzugsweise einer oder mehrere der Stämme DSMZ 16667, DSMZ 16668, DSMZ 16669, DSMZ 16670, DSMZ 16671, DSMZ 16672 und DSMZ 16673 und besonders bevorzugt zumindest DSM 16671, und/oder einer wie oben beschriebenen Mutante oder abgeleiteten Zelllinie, in einer wie oben beschriebenen postexponentiellen Wachstumsphase in einem ersten Schritt in einer Suspension erwärmt werden von einer Ausgangstemperatur unter 40°C auf eine Pasteurisierungstemperatur von 78 bis 80°C, vorzugsweise 80°C, mit einer Temperaturänderung von 0,8 bis 1,2°C, vorzugsweise von 1°C, und anschließend
(ii) die Suspension 25 bis 35 min und bevorzugt 30 min lang auf der Pasteurisierungstemperatur gehalten wird, und anschließend
(iii) die Suspension auf eine Endtemperatur unter 40°C mit einer Temperaturänderung von 0,8 bis 1,2°C, vorzugsweise von 1°C, abgekühlt wird, anschließend wie oben beschrieben mit Wasser gewaschen und durch Zentrifugieren auf einen Trockengehalt von 10 bis 30 Gew.-%, vorzugsweise 18 bis 22 Gew.-%, eingestellt wird, und anschließend
(iv) die Suspension mit einem Träger zum Herstellen einer Sprühtrocknungsmischung versetzt wird, wobei der Träger ausgewählt ist aus Natriumsulfat, Kaliumsulfat und Calciumsulfat und Mischungen zweier oder mehrerer dieser und besonders bevorzugt Natriumsulfat ist, und wobei die Menge an Träger so gewählt wird, dass dass der Trockengehalt der Sprühtrocknungsmischung 10 bis 30 Gew.-% beträgt, und anschließend
(v) die in Schritt (iv) erhaltene, mit Träger versehene Suspension sprühgetrocknet wird bei einer Gaseintrittstemperatur eines zur Trocknung eingesetzten Sprühtrocknungsgases von 180 bis 250°C und einer Gasaustrittstemperatur aus dem zur Trocknung vorgesehenen Bereich, üblicherweise einem Sprühturm, von 70 bis 100°C, vorzugsweise 85°C, wobei die durchschnittliche Verweilzeit des zu trocknenden Gutes im Einwirkbereich des Sprühtrocknungsgases 10 bis 60 Sekunden, vorzugsweise 20 bis 40 Sekunden beträgt.

Dieses Verfahren verwirklicht die oben beschriebenen Vorteile der Erfindung.

Die Lactobacillus-Zubereitung mit spezifischer Bindungsfähigkeit für Streptococcus mutans, die erhältlich oder erhalten ist durch ein Verfahren mit den Schritten i) bis iii) und optional iv) bis v) wird vorzugsweise eingearbeitet in ein Produkt zur Anwendung am menschlichen Körper. Bei Anwendung am menschlichen Körper, insbesondere durch Einbringen in die Mundhöhle und/oder durch Inkontaktbringen mit Zähnen, kann eine Kariesprophylaxe mit den erfindungsgemäßen Zubereitungen tatsächlich erfolgen.

Zweckmäßigerweise enthalten die Produkte die Zubereitung in einer zum Binden an S. mutans und/oder zum Erreichen eines antikariogenen Effekts ausreichenden Menge. Diese Menge hängt ab von der Art des jeweiligen Produktes sowie von seiner Galenik. Insbesondere hängt die Menge auch ab von dem jeweils gewünschten Ausmaß der möglichen Bindung an S. mutans bzw. des möglichen antikariogenen Effekts. Der Fachmann kann durch Routineuntersuchungen je nach Produkt leicht diejenige Menge an erfindungsgemäßer Zubereitung ermitteln, die in Ansehung des gewünschten Effektmaßes, der Produktart und der Galenik ausreichend ist. Insbesondere wird der Fachmann berücksichtigen, dass ein Produkt mit der erfindungsgemäßen Zubereitung beschichtet oder mit dieser Zubereitung durchmischt werden kann.

Das Produkt kann ausgewählt werden aus der Gruppe bestehend aus Nahrungsmittel-, Genussmittel-, Getränke-, Halbfertig-, Mundhygiene-, kosmetischen und pharmazeutischen Produkten. Entsprechende Produkte sind in der WO 2006/027265 A1 beschrieben.

Weitere Produkte sind Kaugummi, Zahnpasta, Mundspülmittel und Bonbons.

Die Erfindung wird nachfolgend anhand der Beispiele näher beschrieben, ohne dass die Beispiele den Schutzbereich der Ansprüche einschränken sollen.

### Beispiel 1: Allgemeines Herstellverfahren

Lactobacillus-Zellen mit spezifischer Bindungsfähigkeit an Streptococcus mutans, wobei das spezifische Binden
a) widerstandsfähig gegen Wärmebehandlung und/oder
b) widerstandsfähig gegen Proteasebehandlung und/oder
c) calziumabhängig ist und/oder
d) in einem pH-Bereich zwischen 4,5 und 8,5 stattfindet, und/oder
e) in Gegenwart von Speichel erfolgt,
werden in einem geeigneten wässrigen Nährmedium bei 37°C kultiviert. Ein geeignetes Nährmedium enthält Glucose, Hefeextrakt, Tween 80 und Salze. Das Kultivieren erfolgt im Fed-Batch-Betrieb als wässrige Suspension.

Sobald der Glucosegehalt des Nährmediums unter 1 mM abgesunken ist, oder bis zu 1 Stunde nach diesem Zeitpunkt, wird die Suspension auf eine Pasteurisierungstemperatur von 75 bis 85°C, bevorzugt 78 bis 80°C erwärmt. Dabei wird die Suspension erwärmt mit einer Temperaturänderung von 0,5 bis 1,2°C/min, vorzugsweise 0,8 bis 1,2°C/min.

Die erwärmte Suspension wird 20 bis 40 Minuten, vorzugsweise 25 bis 35 Minuten und besonders bevorzugt 30 Minuten, auf der Pasteurisierungstemperatur gehalten.

Anschließend wird die Suspension auf unter 40°C abgekühlt. Dabei beträgt die Temperaturänderung 0,5 bis 2°C/min, vorzugsweise 0,8 bis 1,2°C/min.

Die gekühlte Suspension wird anschließend durch Zentrifugation auf einen Trockengehalt von 10 bis 30 Gew.-% aufkonzentriert. Das so erhaltene Konzentrat wird in Wasser resuspendiert und erneut durch Zentrifugation auf einen Trockengehalt von 10 bis 30 Gew.-% aufkonzentriert. Das Resuspendieren und Aufkonzentrieren wird insgesamt bis zu 8 Mal durchgeführt. Am Ende wird eine gewaschene Masse mit 10 bis 30 Gew.-% Trockengehalt erhalten.

Diese Masse besitzt noch immer die Fähigkeit zum spezifischen Binden an Streptococcus mutans wie eingangs im Beispiel beschrieben. Die Masse kann als solche eingearbeitet werden in Nahrungsmittel, Genussmittel, Getränke, Halbfertigprodukte, Mundhygieneprodukte, kosmetische oder pharmazeutische Produkte, um das jeweilige Produkt einzurichten zum Bekämpfen von Karies, oder um dessen bereits anderweitig vorhandene Fähigkeit zum Bekämpfen von Karies zu unterstützen.

### Beispiel 2: Herstellen einer Mundpflegezubereitung (nicht beausprucht)

Eine Kultur von L. paracasei oder L. rhamnosus mit der erfindungsgemäß definierten spezifischen Bindungsfähigkeit an Streptococcus mutans wird wie in Beispiel 1 beschrieben kultiviert, pasteurisiert und gewaschen. Die Pasteurisierungstemperatur beträgt 80°C, die Pasteurisierungszeit 30 min. Die Temperaturänderung beträgt jeweils 0,8 bis 1,2°C/min. Die nach dem ersten Zentrifugieren erhaltene, aufkonzentrierte Suspension wird durch Wasserzugabe resuspendiert auf einen Trockengehalt von 5 Gew.-% der Resuspension. Die so erhaltene Resuspension wird erneut abzentrifugiert bis auf einen Trockengehalt von 20 Gew.-%.

Die so erhaltene gewaschene und konzentrierte Masse wird mit einem wässrigen Träger und Aromen versetzt. Man erhält so eine Mundspülung zum Bekämpfen von Karies.

Auf gleiche Weise werden gewaschene und konzentrierte Massen hergestellt von DSMZ 16667, DSMZ 16668, DSMZ 16669, DSMZ 16670, DSMZ 16671, DSMZ 16672 bzw. DSMZ 16673. Diese Massen werden jeweils ebenfalls mit einem wässrigen Träger und Aromen versetzt, um jeweils eine Mundspülung zum Bekämpfen von Karies zu erhalten.

Anstelle einer Mundspülung werden Kaugummis, Zahnpasten und Bonbons hergestellt zum Bekämpfen von Karies durch Einarbeiten der jeweiligen gewaschenen und konzentrierten Massen in Kaugummigrundmasse, Zahnpastagrundmasse und Bonbongrundmasse sowie Zugeben gewünschter Aromen.

### Beispiel 3: Allgemeines Sprühtrocknungsverfahren

Es wird eine Sprühtrocknungs-Grundlösung hergestellt, indem eine wässrige, 10 bis 30%ige Alkali- und/oder Erdalkalisulfatlösung hergestellt wird. Die gemäß Beispiel 1 erhaltene gewaschene und konzentrierte Masse wird in einer vierfachen Menge der Sprühtrocknungs-Grundlösung zum Bilden einer Sprühtrocknungsmischung resuspendiert, so dass aus 100 Volumeneinheiten der Masse 500 Volumeneinheiten Sprühtrocknungsmischung erhalten werden.

Die Sprühtrocknungsmischung wird sprühgetrocknet durch Eintragen in einen Sprüht trocknungsturm. Der Sprühtrocknungsturm wird beaufschlagt mit einem Sprühtrocknungsgas, wobei das Sprühtrocknungsgas eine Eintrittstemperatur in den Sprühtrocknungsturm von 180 bis 250°C und eine Austrittstemperatur aus dem Sprühtrocknungsturm von 70 bis 100°C besitzt. Die Sprühtrocknungsmischung besitzt eine mittlere Verweilzeit von höchstens 60 Sekunden im Sprühtrocknungsturm.

Das erhaltene sprühgetrocknete Gut besitzt noch immer die Fähigkeit zum spezifischen Binden an Streptococcus mutans wie eingangs im Beispiel 1 beschrieben. Das erhaltene sprühgetrocknete Gut kann als solches eingearbeitet werden in Nahrungsmittel, Genussmittel, Getränke, Halbfertigprodukte, Mundhygieneprodukte, kosmetische oder pharmazeutische Produkte, um das jeweilige Produkt einzurichten zum Bekämpfen von Karies, oder um dessen bereits anderweitig vorhandene Fähigkeit zum Bekämpfen von Karies zu unterstützen.

### Beispiel 4: Herstellen einer weiteren Mundpflegezubereitung (nicht beausprucht)

Eine Kultur von L. paracasei oder L. rhamnosus mit der erfindungsgemäß definierten spezifischen Bindungsfähigkeit an Streptococcus mutans wird wie in Beispiel 1 beschrieben kultiviert, pasteurisiert und gewaschen. Die Pasteurisierungstemperatur beträgt 80°C, die Pasteurisierungszeit 30 min. Die Temperaturänderung beträgt jeweils 0,8 bis 1,2°C/min. Die nach dem ersten Zentrifugieren erhaltene, aufkonzentrierte Suspension wird durch Wasserzugabe resuspendiert auf einen Trockengehalt von 5 Gew.-% der Resuspension. Die so erhaltene Resuspension wird erneut abzentrifugiert bis auf einen Trockengehalt von 20 Gew.-%.

Anschließend wird die Resuspension in einem Sprühtrocknungsverfahren gemäß Beispiel 3 sprühgetrocknet. Die Sprühtrocknungs-Grundlösung ist eine wässrige 20 %ige Natriumsulfatlösung. Die Eintrittstemperatur des Sprühtrocknungsgases in den Sprühturm beträgt 180-250°C. Die Austrittstemperatur des Sprühtrocknungsgases aus dem Sprühturm beträgt 70-100°C. Die mittlere Verweilzeit der Sprühtrocknungsmischung im Sprühturm beträgt 10 Sekunden.

Es wird ein sprühgetrocknetes Gut erhalten. Das sprühgetrocknete Gut wird mit einem wässrigen Träger und Aromen versetzt. Man erhält so eine Mundspülung zum Bekämpfen von Karies.

Auf gleiche Weise wird sprühgetrocknetes Gut hergestellt von DSMZ 16667, DSMZ 16668, DSMZ 16669, DSMZ 16670, DSMZ 16671, DSMZ 16672 bzw. DSMZ 16673.

Dieses Gut wird jeweils ebenfalls mit einem wässrigen Träger und Aromen versetzt, um jeweils eine Mundspülung zum Bekämpfen von Karies zu erhalten.

Anstelle einer Mundspülung werden Kaugummis, Zahnpasten und Bonbons hergestellt zum Bekämpfen von Karies durch Einarbeiten des jeweiligen Gutes in Kaugummigrundmasse, Zahnpastagrundmasse und Bonbongrundmasse sowie Zugeben gewünschter Aromen.

## Patentansprüche

1. Verfahren zum Herstellen einer nichtlebenden Lactobacillus-Zubereitung mit spezifischer Bindungsfähigkeit für Streptococcus mutans, umfassend die Schritte:
i) Erwärmen einer Suspension von Zellen eines Lactobacillus oder einer Mischung von Lactobacillen, mit spezifischer Bindungsfähigkeit an Streptococcus mutans, wobei das spezifische Binden
a) widerstandsfähig gegen Wärmebehandlung und/oder
b) widerstandsfähig gegen Proteasebehandlung und/oder
c) calziumabhängig ist und/oder
d) in einem pH-Bereich zwischen 4,5 und 8,5 stattfindet, und/oder
e) in Gegenwart von Speichel erfolgt,
von einer Ausgangstemperatur unter 40°C auf eine Pasteurisierungstemperatur von 75 bis 85°C mit einer Temperaturänderung von 0,5 bis 2°C/min.,
ii) Halten der erwärmten Suspension auf der Pasteurisierungstemperatur über einen Zeitraum von 20 bis 40 min.,
iii) Abkühlen der in Schritt ii) gehaltenen Suspension auf eine Endtemperatur unter 40°C mit einer Temperaturänderung von 0,5 bis 2°C/min.

2. Verfahren nach Anspruch 1, **dadurch gekennzeichnet, dass** die Lactobacillus-Zellen ausgewählt sind aus Zellen von Lactobacillus paracasei, Lactobacillus rhamnosus, oder einer Mischung derselben.

3. Verfahren nach Anspruch 2, **dadurch gekennzeichnet, dass** die Lactobacillus-Zellen ausgewählt sind aus Zellen von Lactobacillus paracasei oder Lactobacillus rhamnosus, vorzugsweise mit einer der DSMZ-Nummern DSMZ 16667, DSMZ 16668, DSMZ 16669, DSMZ 16670, DSMZ 16671, DSMZ 16672 und DSMZ 16673, oder einer Mutante oder einer abgeleiteten Zelllinie, wobei die Mutante oder abgeleitete Zelllinine die Fähigkeit zum spezifischen Binden an Streptococcus mutans bewahrt hat.

4. Verfahren nach einem der vorherigen Ansprüche, **dadurch gekennzeichnet, dass** der Lactobacillus eine spezifische Bindungsfähigkeit für Streptococcus mutans Serotyp c (DSMZ 20523) und/oder Serotyp e (NCTC 10923) und/oder Serotyp f (NCTC 11060) besitzt.

5. Verfahren nach einem der vorherigen Ansprüche, **dadurch gekennzeichnet, dass** in Schritt i) die Pasteurisierungstemperatur 78 bis 80°C beträgt.

6. Verfahren nach einem der vorherigen Ansprüche, **dadurch gekennzeichnet, dass** die Temperaturänderung in Schritt i) und/oder iii) 0,8 bis 1,2°C/min. beträgt.

7. Verfahren nach einem der vorherigen Ansprüche, **dadurch gekennzeichnet, dass** in Schritt i) das Medium der Suspension maximal 10mmol Glucose/I enthält.

8. Verfahren nach einem der vorherigen Ansprüche, ferner umfassend die Schritte:
iv) Versetzen der in Schritt iii) erhaltenen Suspension mit einem Träger zum Herstellen einer Sprühtrocknungs-Mischung, wobei der Träger ausgewählt ist aus Alkali- und Erdalkali-Sulfaten, und wobei die Menge so gewählt wird, dass der Anteil an Trockensubstanz an der Sprühtrocknungs-Suspension 10 bis 30 Gew.-% beträgt,
v) Sprühtrocknen der ein Schritt iv) erhaltenen Sprühtrocknungs-Mischung.

9. Verfahren nach Anspruch 8, **dadurch gekennzeichnet**, bei einer Gaseintrittstemperatur eines zur Trocknung eingesetzten Sprühtrocknungsgases von 180 bis 250°C und einer Austrittstemperatur Gasaustrittstemperatur aus dem zur Trocknung vorgesehenen Bereich, üblicherweise einem Sprühturm, von 70 bis 100°C, vorzugsweise 85°C, wobei die durchschnittliche Verweilzeit des zu trocknenden Gutes im Einwirkbereich des Sprühtrocknungsgases 10 bis 60 Sekunden, vorzugsweise 20 bis 40 Sekunden beträgt.

## Claims

1. A method for producing a nonlive Lactobacillus composition with specific binding ability for Streptococcus mutans, comprising the following steps:
i) warming a suspension of cells of a Lactobacillus or a mixture of Lactobacilli with specific binding ability to Streptococcus mutans, where the specific binding
a) is resistant to heat treatment and/or
b) is resistant to protease treatment and/or
c) is calcium-dependent and/or
d) takes place in a pH range of between 4.5 and 8.5 and/or
e) takes place in the presence of saliva,
from a starting temperature of below 40°C to a pasteurization temperature of 75 to 85°C with a temperature change of 0.5 to 2°C/min,
ii) holding the warm suspension at the pasteurization temperature over a period of from 20 to 40 min,
iii) cooling the suspension held in step ii) to a final temperature of below 40°C, with a temperature change of 0.5 to 2°C/min.

2. The method according to claim 1, wherein the Lactobacillus cells are selected from among cells of strains of Lactobacillus paracasei, Lactobacillus rhamnosus or a mixture of these.

3. The method according to claim 2, wherein the Lactobacillus cells are selected from among cells of Lactobacillus paracasei or Lactobacillus rhamnosus, preferably with one of the DSMZ numbers DSMZ 16667, DSMZ 16668, DSMZ 16669, DSMZ 16670, DSMZ 16671, DSMZ 16672 and DSMZ 16673, or a mutant or a derived cell line, the mutant or derived cell line having retained the ability of specifically binding to Streptococcus mutans.

4. The method according to any of the preceding claims, wherein the Lactobacillus cells have a specific binding ability for Streptococcus mutans Serotype c (DSMZ 20523) and/or Serotype e (NCTC 10923) and/or Serotype F (NCTC 11060).

5. The method according to any of the preceding claims, wherein the pasteurization temperature in step i) is from 78 to 80°C.

6. The method according to any of the preceding claims, wherein the temperature change in step i) and/or iii) is from 0.8 to 1.2°C/min.

7. The method according to any of the preceding claims, wherein the medium of the suspension in step i) comprises not more than 10 mmol glucose/l.

8. The method according to any of the preceding claims, furthermore comprising the following steps:
iv) treating the suspension obtained in step iii) with a carrier for preparing a spray-drying mixture, the carrier being selected from among alkali metal sulfates and alkaline-earth metal sulfates, the amount of carrier being chosen such that the dry-matter content of the spray-drying suspension amounts to 10 to 30% by weight,
v) spray-drying of the spray-drying mixture obtained in step iv).

9. The method according to claim 8, wherein spray-drying is effected at a gas inlet temperature of a spray-drying gas employed for drying of from 180 to 250°C and a gas outlet temperature from the zone provided for drying, usually a spray tower, of from 70 to 100°C, preferably 85°C, the average residence time in the reaction zone of the spray-drying gas of the material to be dried being from 10 to 60 seconds, preferably from 20 to 40 seconds.

## Revendications

1. Procédé pour la production d'une préparation de *Lactobacillus* non vivant, à aptitude de liaison spécifique à *Streptococcus mutans,* comprenant les étapes :
i) chauffage d'une suspension de cellules d'un *Lactobacillus* ou d'un mélange de bacilles lactiques, à aptitude de liaison spécifique à *Streptococcus mutans,* la liaison spécifique étant
a) résistante au traitement par la chaleur et/ou
b) résistante au traitement par des protéases et/ou
c) dépendante du calcium et/ou
d) ayant lieu dans un intervalle de pH compris entre 4,5 et 8,5, et/ou
e) s'effectuant en présence de salive,
d'une température initiale inférieure à 40 °C à une température de pasteurisation de 75 à 85 °C avec une variation de température de 0,5 à 2 °C/min.,
ii) maintien à la température de pasteurisation de la suspension chauffée, pendant une durée de 20 à 40 min.,
iii) refroidissement de la suspension maintenue dans l'étape ii), jusqu'à une température finale inférieure à 40 °C avec une variation de température de 0,5 à 2 °C/min.

2. Procédé selon la revendication 1, **caractérisé en ce que** les cellules de *Lactobacillus* sont choisies parmi des cellules de *Lactobacillus paracasei, Lactobacillus rhamnosus,* ou d'un mélange de ceux-ci.

3. Procédé selon la revendication 2, **caractérisé en ce que** les cellules de *Lactobacillus* sont choisies parmi des cellules de *Lactobacillus paracasei* ou *Lactobacillus rhamnosus,* de préférence portant l'un des numéros DSMZ DSMZ 16667, DSMZ 16668, DSMZ 16669, DSMZ 16670, DSMZ 16671, DSMZ 16672 et DSMZ 16673, ou d'un mutant ou d'une lignée cellulaire dérivée, le mutant ou la lignée cellulaire dérivée ayant conservé l'aptitude à la liaison spécifique à *Streptococcus mutans.*

4. Procédé selon l'une quelconque des revendications précédentes, **caractérisé en ce que** le *Lactobacillus* possède une aptitude à la liaison spécifique pour *Streptococcus mutans* sérotype c (DSMZ 20523) et/ou sérotype e (NCTC 10923) et/ou sérotype f (NCTC 11060).

5. Procédé selon l'une quelconque des revendications précédentes, **caractérisé en ce que** dans l'étape i) la température de pasteurisation vaut de 78 à 80 °C.

6. Procédé selon l'une quelconque des revendications précédentes, **caractérisé en ce que** la variation de température dans l'étape i) et/ou l'étape iii) vaut de 0,8 à 1,2 °C/min.

7. Procédé selon l'une quelconque des revendications précédentes, **caractérisé en ce que** dans l'étape i) le milieu de la suspension contient au maximum 10 mmoles de glucose/l.

8. Procédé selon l'une quelconque des revendications précédentes, comprenant en outre les étapes :
iv) addition à la suspension obtenue dans l'étape iii) d'une substance de support pour la préparation d'un mélange pour séchage par atomisation, la substance de support étant choisie parmi des sulfates de métaux alcalins et de métaux alcalino-terreux, et la quantité étant choisie de manière que la teneur en substance sèche de la suspension pour séchage par atomisation soit de 10 à 30 % en poids,
v) séchage par atomisation du mélange pour séchage par atomisation obtenu dans l'étape iv).

9. Procédé selon la revendication 8, **caractérisé en ce qu'**on effectue le séchage par atomisation à une température d'entrée de gaz de 180 à 250 °C d'un gaz de séchage par atomisation utilisé pour le séchage et à une température de sortie de gaz hors de la zone prévue pour le séchage, habituellement d'une tour de pulvérisation, de 70 à 100 °C, de préférence de 85 °C, le temps de séjour moyen du produit à sécher dans la zone d'action du gaz de séchage par atomisation étant de 10 à 60 secondes, de préférence de 20 à 40 secondes.
